# EUROPEAN PATENT APPLICATION

(11) **EP 4 644 571 A1**
(43) Date of publication of application: **05.11.2025**
(21) Application number: 23912909.1
(22) Date of filing: 27.12.2023
(51) Int. Cl.: C12Q 1/6886

(54) **METHOD FOR DETECTION OF LUNG CANCER USING LUNG CANCER-SPECIFIC METHYLATION MARKER GENE**

(30) Priority: 27.12.2022 KR 20220185602
(71) Applicant: Genomictree, Inc., Daejeon 34027 (KR)
(72) Inventor: OH, Taejeong, Daejeon 34127 (KR); AN, Sungwhan, Daejeon 34125 (KR)
(74) Representative: Mewburn Ellis LLP
(86) International application number: PCT/KR2023/021645
(87) International publication number: WO 2024/144235

(57) **Abstract**

The present invention relates to novel use of a paired related homeobox 1 (*PRRX1*) gene and/or an ATP binding cassette subfamily C member 9 (*ABCC9*) gene as lung cancer-specific methylation marker(s) and, in particular, to a composition for diagnosing lung cancer by detecting methylation by using the PRRX1 or ABCC9 gene as a biomarker, a kit comprising same, and a method for providing information for lung cancer diagnosis.

## Description

### [Technical Field]

The present invention relates to the novel use of *PRRX1* (paired related homeobox 1) and/or *ABCC9* (ATP binding cassette subfamily C member 9) genes as lung cancer-specific methylation markers, and more particularly, to a composition for diagnosing lung cancer by detecting methylation using the *PRRX1* or *ABCC9* gene as a biomarker, a kit including the same, and a method of providing information for lung cancer diagnosis.

### [Background Art]

In addition to A, C, G, and T, the genomic DNA of mammalian cells contains a fifth base, 5-methylcytosine (5-mC), which has a methyl group attached to the fifth carbon of the cytosine ring. 5-mC is always attached to C of a CG dinucleotide (5'-mCG-3'), and this CG is often denoted as CpG. C in CpG is mostly methylated, with a methyl group attached thereto. Methylation of CpG inhibits the expression of repetitive sequences in the genome, such as Alu or transposons, and CpG is the site where extragenic changes most frequently occur in mammalian cells. 5-mC of the CpG is naturally converted into T by deamination, and accordingly, CpG in the mammalian genome appears only with a frequency of 1%, which is much lower than a normal frequency (1/4 x 1/4 = 6.25%).

There is a region in which CpGs are exceptionally dense, which is called a CpG site (CpG island). The CpG site is 0.2 to 3 kb in length, and is a highly concentrated region in which the distribution percentage of C and G bases is greater than 50% and the distribution percentage of CpG is 3.75% or more. About 45,000 CpG sites appear throughout the entire human genome, and are intensively found in the promoter region that regulates gene expression. In fact, CpG sites appear in the promoters of housekeeping genes, which account for about half of human genes (Cross, S. et al., Curr. Opin. Gene Develop., 5:309, 1995). Abnormal DNA methylation is known to occur primarily in the 5' regulatory region of the relevant gene, thereby reducing expression thereof.

Meanwhile, in somatic cells of normal humans, the CpG islands in the promoter regions of housekeeping genes are not methylated, but imprinted genes and inactivated genes on the X chromosome are methylated so as to prevent the expression thereof during development.

During oncogenesis, methylation occurs in promoter CpG islands, and expression of the corresponding gene is impaired. In particular, when methylation occurs in the regulatory region CpG islands of tumor suppressor genes, which regulate cell cycles or apoptosis, repair DNA, participate in cell adhesion and cell-to-cell interaction, and suppress invasion and metastasis, the expression and function of these genes are blocked, just like mutations in coding sequences, resulting in promoted development and progression of cancer. In addition, partial methylation may appear on CpG islands with aging.

Methylation of the regulatory regions of tumor-related genes is an important indicator of cancer, and therefore may be used in various ways, such as for cancer diagnosis and early diagnosis, prediction of oncogenesis risk, prediction of cancer prognosis, follow-up after treatment, prediction of response to anticancer therapy, and the like. Recently, there have been active attempts to investigate promoter methylation of tumor-related genes in the blood, sputum, saliva, stool, urine, etc. and to use the results thereof in the treatment of various types of cancer (Ahlquist, D.A. et al., Gastroenterol., 119:1219, 2000).

Against this technical background, the inventors of the present application have made great efforts to develop an effective lung cancer-specific methylation marker capable of early diagnosis, prediction of oncogenesis risk or cancer prognosis, etc., and thus ascertained that the *PRRX1* or *ABCC9* gene is lung cancer-specific, thereby culminating in the present invention.

### [Disclosure]

It is an object of the present invention to provide a lung cancer-specific methylation biomarker and a composition for diagnosing lung cancer using the same.

It is another object of the present invention to provide a method of diagnosing lung cancer using the lung cancer-specific methylation biomarker.

It is still another object of the present invention to provide the use of the lung cancer-specific methylation biomarker for the diagnosis of lung cancer.

It is yet another object of the present invention to provide a kit for diagnosing lung cancer including the composition.

It is still yet another object of the present invention to provide a method of providing information for lung cancer diagnosis using a methylated lung cancer marker gene as the lung cancer-specific methylation biomarker.

In order to accomplish the above objects, the present invention provides a composition for diagnosing lung cancer containing a substance capable of detecting CpG island methylation of a *PRRX1* (paired related homeobox 1) gene and/or an *ABCC9* (ATP binding cassette subfamily C member 9) gene.

In addition, the present invention provides a method of diagnosing lung cancer, including administering, to a patient, a substance capable of detecting CpG island methylation of a *PRRX1* (paired related homeobox 1) gene and/or an *ABCC9* (ATP binding cassette subfamily C member 9) gene, or treating a sample of a patient with a material capable of detecting CpG island methylation of a *PRRX1* (paired related homeobox 1) gene and/or an *ABCC9* (ATP binding cassette subfamily C member 9) gene.

Furthermore, the present invention provides the use of a substance capable of detecting CpG island methylation of a *PRRX1* (paired related homeobox 1) gene and/or an *ABCC9* (ATP binding cassette subfamily C member 9) gene for the manufacture of a composition for diagnosing lung cancer.

The present invention also provides a kit for diagnosing lung cancer including the composition described above.

The present invention also provides a method of providing information for lung cancer diagnosis, including treating a sample with a substance capable of detecting CpG island methylation of a *PRRX1* (paired related homeobox 1) gene and/or an *ABCC9* (ATP binding cassette subfamily C member 9) gene.

### [Description of Drawings]

FIG. 1 shows a CpG microarray analysis design for discovering novel methylation biomarker candidates in lung cancer tissue;
FIG. 2 shows a process of discovering novel methylation biomarker candidates through statistical analysis in lung cancer tissue;
FIG. 3 shows the methylation status of two biomarker candidate genes in a lung cancer cell line (A) and tissue (B) measured by pyrosequencing;
FIG. 4 shows the methylation status of two biomarker genes in bronchial lavage fluids of lung cancer patients and benign lung disease patients measured by methylation-specific real-time PCR, and the sensitivity and specificity for lung cancer diagnosis measured by ROC analysis; and
FIG. 5 shows the methylation status of the *PRRX1* gene in the blood of healthy persons and lung cancer patients measured by methylation-specific real-time PCR.

### [Mode for Invention]

Unless otherwise defined, all technical and scientific terms used herein have the same meanings as typically understood by those skilled in the art to which the present invention belongs. In general, the nomenclature used herein is well known in the art and is typical.

An aspect of the present invention relates to the novel use of lung cancer-specific methylation genes *PRRX1* (paired related homeobox 1) and/or *ABCC9* (ATP binding cassette subfamily C member 9), namely the use thereof for lung cancer diagnosis, and more particularly, to a composition for lung cancer diagnosis containing a substance capable of detecting CpG island methylation of a *PRRX1* (paired related homeobox 1) gene and/or an *ABCC9* (ATP binding cassette subfamily C member 9) gene.

In addition, the present invention provides a method of diagnosing lung cancer, including administering, to a patient, a substance capable of detecting CpG island methylation of a *PRRX1* (paired related homeobox 1) gene and/or an *ABCC9* (ATP binding cassette subfamily C member 9) gene, or treating a sample of a patient with a substance capable of detecting CpG island methylation of a *PRRX1* (paired related homeobox 1) gene and/or an *ABCC9* (ATP binding cassette subfamily C member 9) gene.

Furthermore, the present invention provides the use of a substance capable of detecting CpG island methylation of a *PRRX1* (paired related homeobox 1) gene and/or an *ABCC9* (ATP binding cassette subfamily C member 9) gene for the manufacture of a composition for diagnosing lung cancer.

Another aspect of the present invention relates to a method of providing information for lung cancer diagnosis, including treating a sample with a substance capable of detecting CpG island methylation of a *PRRX1* (paired related homeobox 1) gene and/or an *ABCC9* (ATP binding cassette subfamily C member 9) gene.

The present invention provides information for lung cancer diagnosis by measuring methylation of *PRRX1* and/or *ABCC9* genes, which are specifically methylated in lung cancer cells. In addition, it has been confirmed that these novel markers are highly useful for lung cancer diagnosis because lung cancer may be diagnosed with excellent sensitivity and specificity by detecting CpG island methylation thereof.

As used herein, the term "methylation" refers to modification of cytosine into 5-methylcytosine (5-mC) in which a methyl group is attached to the fifth carbon of a cytosine base ring. 5-methylcytosine is always attached only to C of CG dinucleotide (5'-mCG-3'), and this CG is commonly denoted as CpG. Methylation of CpG inhibits the expression of repetitive sequences in the genome, such as Alu or transposons, and CpG is the site where extragenic changes most frequently occur in mammalian cells. 5-mC of this CpG is naturally converted into T by deamination, and thus, CpG in the mammalian genome appears only with a frequency of 1%, which is much lower than a normal frequency (1/4 x 1/4 = 6.25%).

There is a region in which CpGs are exceptionally dense, which is called a CpG island. The CpG island is 0.2 to 3 kb in length, and is a highly concentrated site in which the distribution percentage of C and G bases is greater than 50% and the distribution percentage of CpG is 3.75% or more. About 45,000 CpG islands appear throughout the entire human genome, and are intensively found in the promoter region that regulates gene expression. CpG islands actually appear in promoters of housekeeping genes, which account for about half of human genes.

The CpG island may be located at any region of the *PRRX1* or *ABCC9* gene. Particularly, the CpG island may be located in the promoter region of the *PRRX1* or *ABCC9* gene, or may be present in the upstream or downstream region of the *PRRX1* or *ABCC9* gene. Particularly, the CpG island may be present in an intron, exon, or enhancer of the *PRRX1* or *ABCC9* gene, but the present invention is not limited thereto.

According to a certain embodiment of the present invention, lung cancer-specific hypermethylation was confirmed to occur in the CpG island in the promoter region of the *PRRX1* or *ABCC9* gene. Based thereon, the CpG island may be located in the promoter of the *PRRX1* or *ABCC9* gene.

The *PRRX1* gene may include, for example, the sequence of SEQ ID NO: 1. The CpG island of the *PRRX1* gene may be present, for example, at a position -1,505 to -1,299 from the transcription initiation site (+1) in the sequence of SEQ ID NO: 1. Particularly, the CpG island of the *PRRX1* gene may be present at positions -1,505, -1,411, and -1,343 from the transcription initiation site (+1) in the sequence of SEQ ID NO: 1.

[SEQ ID NO: 1]

GTGTAAATGAGGATAGAGAGTGCGAAAGGAGGGAGAGGCGAGGAGCTGCT
CTATTTCTAGGGAGGTTTTGGGGAGACTGATCAGCTCCAAGGACAGACC
GCTGGGAAGGGAAAAACGGCCCACATCGAACTGGATGCCGGATGGAAACC
TCTCTGCGCTATTAGACTGCGTCCAGTACAGCAGATGGCACGAGCACGT
GCGGCGCTCAGCTTAGGCTCTCGGAGGCAGCTGAGTTGGAAATCCCGAC
GGAAAGCACCCACAAGCTCCCACTCTGCGCTGGCCCACCCGCGTGCACGCC
CACCCCCCACGCGCGTCCCTGGCTCAGAAGCGCACAGATGTTTACTGCTTAG
AGCCGGTACCGCTGGGGAGATCGAGCGACTTGCGCGGCGCACAGTGCGGCG
CTGGCAGGGCTCTGGGCTCCCGGTCGGGGGTTCGAGCGGCCAAGGGATGGG
GGTGGGGGCGGGGAGAGTGGGGGGAGGGCGAAAGACCGCCGAGAGGAGG
GGGGAGTGGGTGGACTAATGATGAAAAAGTCTCCTCCATCCCAGTTCCTTA
ATTAAATGCATGGAAAGAACCGAGGCGAGCACATCTGGTTTCAATCTACAG
CCCTTTGATGGCATCAAATGTTCTTTTCCCAGATCAGGGCTGGAAGTTCTGG
GCTAACTATGGCCGTTTGGAGCCCAGAAACCATTTACACACACTCGTACCCT
TCTTTCTCTCCAGTCGAGCCTCTTGACTATAGGACGAAAAAAAAAAAAAGT
CTAGCAATCAAGGGAGTGCGGCAGTACGGATGCGTGTCTGTGTGTGTGAGT
GCGCGTTTAAAGAACATAAAACGCCACAAATAAGCACTTAATATTTTACTG
AGTCGTCATACAGTAACTCATTTCTAATGAGACTGTTAAAGCTGTTACCAAA
TTCCAACAGGTCATGAAAAAACTGCTCAGCACCTCTCTGTCTTCTGTCAGGT
TAGGGCTCCGCAGACGGACGACAGAGGATTATCCGTAGACTTAGATGGGGG
TTCCTCCGCCCCTCTCTGTCCTCCTGCCACCACAGCCAGGCTGGACGCAGAA
CCGCACGGAAGCCTCCCCAACTCCTGCCTCTGGGAGTGCTCCTGCCTGCTTC
AAGGAATCTGGGCAGCGGGACCCTCTGGGTTTAGCGTCCTCGCCTGGGGAA
GCAACTTTGGCCTTACCGTAGAAGTTACAAAATTGGCGGGGGTAGGGAGAA
AGATTATAATATATATTTGGAGTCCTTTCTTCCAGTTTAATAAACACGTACTT
TTCTCATTGCCCTAGTCCTGGTATGCCTAATGGCGGGGAAAGCCTCAAATAA
ACTCGGTGCCCTCTGGATCTCCCCAACCTGCCTTTAGTCCTTGCGAGAGGCG
TGAGAGAACTTCCTATTATCATCCCTTTTGTTATTTTTATTACATTATTTAAT
AGGCTCCCTCTACGGAACCAAAAATAGCCTTAATATGCTTACTTTCTCCCTG
TACTCCGCCCCAAACTGTTAGAACAGCAGAAAATTTTTTTTGGGGGGGGC
GGTTTGGGGGAAGGCAGAAGGAGAAGAAAATTTGATTGTGATGGTGGTGG
ATTTTTTCCAAATTT

The *ABCC9* gene may include, for example, the sequence of SEQ ID NO: 2. The CpG island of the *ABCC9* gene may be present, for example, at a position -373 to -220 from the transcription initiation site (+1) in the sequence of SEQ ID NO: 2. Particularly, the CpG island of the *ABCC9* gene may be present, for example, at positions -373 and - 264 from the transcription initiation site (+1) in the sequence of SEQ ID NO: 2.

[SEQ ID NO: 2]
CCATGGGAATCAGCGTCTCACTTTAGGGGACGGTGGGCAAGGGTCG
CCCTTCGATAGCGCGGCGGCTGGTCCACCAGAGAAACCAAGTTTTCTAGG
TCGCCGGGGAAAAGCGGAAAATTAGACTTGAGCCAGCCTTGCTCCTGTGA
GACATTCGTGCACTCATAACCCAGCAGTGGGTGCCTCTACCTAGAATGAGC
CCTTCATTTCCCGGATCCGTTCAGGGGGAAACAGCGAAATCCCCAAATC
CGCTTCCTTACGTGGCTAATAAAGGGGTGCTTACAGAGCATGGAAAAACAA
AACAAAGAAACAAGAATACCTTGAGTGTTTTGACGTTTGAAAACCCGACTC
CGGAGAATCATGGAATCATATTCCAAGTTTCAGTGTCTAATGAAGTTTAAGG
GGACGCCTATTACTTGGGTTTGTAAAATCGCGGCGTCGCGCCCGCCCCAA
GTCAAAGCTGAGCAGAGCAGGGATGGGGAAGGTCTCCGGCCCCAGGCGCG

The genes for methylation biomarkers for lung cancer diagnosis according to the present invention are described below.

| | Probe position* | GenBank No. | Description |
|---|---|---|---|
| *PRRX1* | -1,505, -1,411, -1,343 | NG_031856 | Paired related homeobox 1 |
| *ABCC9* | -373, -264 | NG_012819 | ATP binding cassette subfamily C member 9 |

| | | | |
|---|---|---|---|
| *Sequence distance (bp) from transcription initiation site (+1) | | | |

Particularly, the *PRRX1* gene may include, for example, the sequence of SEQ ID NO: 1. The CpG island of the *PRRX1* gene may be present, for example, at a position -1,505 to -1,299 from the transcription initiation site (+1: **A underlined A** in SEQ ID NO: 1) in the sequence of SEQ ID NO: 1. The *PRRX1* target position may be -1,505 to -1,460 **CTGCTCTATTTCTAGGGAGGTTTTGGGGAGACTGATCAGCTCCAAG,** -1,411 to - 1,366

**GATGGAAACCTCTCTGCGCTATTAGACTGCGTCCAGTACAGCAGAT,** or -1,343 to -1,299 **AGCTTAGGCTCTCGGAGGCAGCTGAGTTGGAAATCCCGACGGAAA** (based on **A underlined A** in the transcription initiation point SEQ ID NO: 1).

Particularly, the *ABCC9* gene may include, for example, the sequence of SEQ ID NO: 2. The CpG island of the *ABCC9* gene may be present, for example, at a position -373 to -220 from the transcription initiation site (+1: **G underlined G** in SEQ ID NO: 2) in the sequence of SEQ ID NO: 2. The *ABCC9* target position may be -373 to -326 **AGAAACCAAGTTTTCTAGGTCGCCGGGGAAAAGCGGAAAATTAGACTT** or -264 to -220 **CTAGAATGAGCCCTTCATTTCCCGGATCCGTTCAGGGGGAAACAG** (based on G **underlined G** in the transcription initiation point SEQ ID NO: 2).

The CpG island methylation may be detected by real-time quantitative amplification, for example, real-time polymerase chain reaction (real-time PCR), and the amount of PCR amplicon in real-time polymerase chain reaction may be detected based on a fluorescence signal. As the real-time polymerase chain reaction progresses, the intensity of the fluorescence signal increases with an increase in the amount of polynucleotide, and an amplification profile curve showing the fluorescence signal intensity depending on the number of amplification cycles is obtained.

In general, the amplification profile curve is divided into a baseline region which shows a fluorescence signal in the background that does not reflect the actual polynucleotide amount, an exponential region in which a fluorescence signal increases with an increase in the amount of polynucleotide product, and a plateau region in which PCR reaches saturation and thus there is no increase in the intensity of the fluorescence signal.

Typically, the fluorescence signal intensity at the transition point from the baseline region to the exponential region, namely at the point when the amount of a PCR amplicon reaches an amount detectable by fluorescence, is referred to as a threshold, and the number of amplification cycles corresponding to the threshold on the amplification profile curve is referred to as a threshold cycle (Ct) value.

By measuring the Ct value, analyzing the standard curve in which the concentration is determined based on the Ct (threshold cycle) value for a standard substance, and confirming the concentration of the amplified gene, methylation-specific sensitivity and/or specificity may be determined.

In one embodiment, the methylation may be detected by any method selected from the group consisting of PCR, methylation-specific PCR, real-time methylation-specific PCR, PCR using a methylated DNA-specific binding protein, PCR using a methylated DNA-specific binding antibody, quantitative PCR, gene chip, sequencing, sequencing by synthesis, and sequencing by ligation.
(1) Methylation-specific PCR: For detection by the methylation-specific PCR, when treated with bisulfite, cytosine in the 5'-CpG-3' region remains as cytosine in case of methylation, and is converted into uracil in case of non-methylation. Therefore, primers corresponding to a region where the 5'-CpG-3' sequence exists may be constructed for the sequence converted after treatment with bisulfite. When PCR is performed using primers, a PCR product is created due to the use of the primers corresponding to the methylated sequence in case of methylation, and methylation may be confirmed by agarose gel electrophoresis. Here, the methylation detection probe may be, but not limited to, TaqMan, Molecular Beacon, a probe having a self-reporting function, or a probe having an energy transfer labeling function.
(2) Real-time methylation-specific PCR: Real-time methylation-specific PCR is a real-time measurement method modified from methylation-specific PCR, and includes treating genomic DNA with bisulfite, designing PCR primers corresponding to the methylated sequence, and performing real-time PCR using the primers. As such, there are two methods: detection using a TaqMan probe complementary to the amplified sequence and detection using SYBR Green. Therefore, real-time methylation-specific PCR is capable of selectively quantitatively analyzing only methylated DNA. As such, a standard curve may be created using an *in vitro* methylated DNA sample, and a gene without 5'-CpG-3' in the sequence may also be amplified as a negative control for standardization, quantitatively analyzing the extent of methylation.
(3) PCR or quantitative PCR and DNA chip assay using methylated DNA-specific binding protein: In the method of PCR or DNA chip using a methylated DNA-specific binding protein, when a protein that specifically binds only to methylated DNA is mixed with DNA, the protein specifically binds only to methylated DNA, so methylated DNA may be selectively isolated.
   In addition, methylation may be measured by quantitative PCR, and methylated DNA isolated using a methylated DNA-specific binding protein may be labeled with a fluorescent dye and hybridized to a DNA chip integrated with complementary probes to thereby measure methylation.
(4) Detection of differential methylation - bisulfite sequencing method: Another method of detecting methylated CpG-containing nucleic acid includes bringing a nucleic acid-containing sample into contact with an agent that modifies unmethylated cytosine and amplifying the CpG-containing nucleic acid in the sample using CpG-specific oligonucleotide primers. Here, the oligonucleotide primers may be characterized by detecting the methylated nucleic acid by distinguishing between modified methylated and unmethylated nucleic acids. The amplification step is optional and desirable, but not essential. This method relies on PCR to distinguish between modified (e.g., chemically modified) methylated DNA and unmethylated DNA.
(5) Bisulfite sequencing method: Another method of detecting methylated CpG-containing nucleic acid includes bringing a nucleic acid-containing sample into contact with an agent that modifies unmethylated cytosine and amplifying the CpG-containing nucleic acid in the sample using methylation-independent oligonucleotide primers. Here, the oligonucleotide primers may be characterized by amplifying the nucleic acid without distinguishing between modified methylated and unmethylated nucleic acids. The amplified product is described in connection with bisulfite sequencing for detection of methylated nucleic acid by the next-generation sequencing method or for sequencing by the Sanger method using sequencing primers.
(6) Examples of the next-generation sequencing method may include a sequencing-by-synthesis method and a sequencing-by-ligation method. These methods are characterized in that, instead of creating a bacterial clone, a single DNA fragment is spatially isolated, amplified *in situ* (clonal amplification), and sequenced. Here, such a method is also called massively parallel sequencing because hundreds of thousands of fragments are read simultaneously.

Basically, a sequencing-by-synthesis method is performed, and a method of obtaining signals by sequentially attaching mono- or di-nucleotides is used, examples of which may include pyrosequencing, Ion Torrent, Solexa, and the like.

Examples of NGS devices based on the sequencing-by-synthesis method include the 454 platform from Roche, the HiSeq platform from Illumina, the Ion PGM platform from Life Technology, and the PacBio platform from Pacific BioSciences. For 454 and Ion PGM, emersion PCR is used as a clonal amplification method, and for HiSeq, bridge amplification is used. The sequencing-by-synthesis method serves to read the sequence by detecting phosphate, protons, or pre-attached fluorescence generated when synthesizing DNA by sequentially attaching one nucleotide at a time. In the sequence detection method, the pyrosequencing method using phosphoric acid is performed for 454, and proton detection is performed for Ion PGM. For HiSeq and PacBio, fluorescence is detected to decode sequences.

Sequencing-by-ligation is a sequencing technique using DNA ligase to identify nucleotides at certain positions in a DNA sequence. Unlike most sequencing techniques using polymerases, the sequencing-by-ligation method does not use a polymerase and is characterized in that DNA ligase does not ligate mismatched sequences. An example thereof is the SOLiD system. In this technique, bases are read two at a time with spacing, which is repeated five times independently through primer reset, so that each base is ultimately read twice in duplicate, increasing accuracy.

In the sequencing-by-ligation method, among the dinucleotide primer sets made of 16 combinations, dinucleotide primers corresponding to the relevant sequence are sequentially ligated, and the combination of these ligations is finally analyzed to thus complete the sequence of the corresponding DNA.

Here, the next-generation sequencing method may be exemplified by a sequencing-by-synthesis method or a sequencing-by-ligation method. The methylated DNA-specific binding protein is, but not limited to, MBD2bt, and the antibody is, but not limited to, a 5'-methyl-cytosine antibody.

Accordingly, the substance capable of detecting CpG island methylation is not limited so long as it is able to detect positive methylation at a high level and frequency specific to lung cancer, and may be, for example, a primer or primer pair capable of amplifying a fragment including a methylated CpG island, a probe capable of hybridizing with the methylated CpG island, a sequencing primer, a sequencing-by-synthesis primer, or a sequencing-by-ligation primer.

The term "primer" as used herein refers to a single-stranded oligonucleotide capable of acting as an initiation point for template-directed DNA synthesis under appropriate conditions (i.e., four different nucleoside triphosphates and polymerase) in a buffer at an appropriate temperature.

The primer may be constructed to have "substantial" complementarity with each strand of the gene locus to be amplified. This means that the primer has sufficient complementarity to hybridize with the corresponding nucleic acid strand under conditions for performing polymerization reaction.

The primer may mean, for example, a region having a length of 5 bp to 50 bp, or 10 bp to 30 bp, for the target gene or any region thereof.

As used herein, the term "complementary binding" means that the primer hybridizes with the corresponding nucleic acid strand under conditions for performing polymerization reaction, forming a duplex structure. Complementary binding may be formed when the complementarity between the paired nucleotide sequences forms a Watson-Crick pair, or even when some non-Watson-Crick base pairs exist.

As used herein, a "probe" is a single-stranded nucleic acid molecule capable of hybridizing, including a sequence substantially complementary to a target nucleic acid sequence.

In hybridization reaction, the conditions used to achieve a certain level of stringency vary depending on the nature of the nucleic acid to be hybridized. For example, the length of the nucleic acid site to be hybridized, the degree of homology, the nucleotide sequence composition (e.g., GC/AT composition ratio), and the type of nucleic acid (e.g., RNA, DNA) are considered in selecting the hybridization conditions. A further consideration is whether the nucleic acid is immobilized, for example on a filter or the like.

Examples of very stringent conditions are as follows: 2X SSC/0.1% SDS at room temperature (hybridization conditions); 0.2X SSC/0.1% SDS at room temperature (low stringency conditions); 0.2X SSC/0.1% SDS at 42°C (moderate stringency conditions); and 0.1X SSC at 68°C (high stringency conditions). The washing process may be performed using any one of these conditions, and, for example, high stringency conditions or each of the above conditions may be used, the conditions may be applied for 10 to 15 minutes each in the order described above, or all or some of the conditions described above may be repeatedly applied. As described above, however, the optimal conditions vary depending on the special hybridization reaction involved, and may be determined through experimentation. Generally, high stringency conditions are used for hybridization of the probe of interest.

In some cases, the primer or probe may be detectably labeled, for example, with a radioisotope, a fluorescent compound, a bioluminescent compound, a chemiluminescent compound, a metal chelate, or an enzyme. Appropriate labeling of the primer or probe is a technique widely known in the art and may be performed by a typical method.

The amount of the amplicon may be detected based on a fluorescence signal. The detection method may include an intercalation method using an intercalator that fluoresces by binding to the double-stranded DNA of the amplicon to which the primer or probe is bound, a method of using an oligonucleotide in which the 5' end is labeled with a fluorescent substance and the 3' end is labeled with a quencher, etc.

In one embodiment, the substance capable of detecting CpG island methylation may be a primer or primer pair capable of amplifying a fragment containing the methylated CpG island and/or a sequencing primer.

In a certain embodiment according to the present invention, PCR and sequencing primers were designed to perform pyrosequencing on the *PRRX1* or *ABCC9* gene. The CpG islands located at positions -1,361, -1,355, -1,351, and - 1,348 from the transcription initiation site (+1) in the sequence of the *PRRX1* gene SEQ ID NO: 1 may be amplified.

Particularly, PCR primers for measuring methylation of the *PRRX1* gene may include primers including the sequence of SEQ ID NO: 3 and the sequence of SEQ ID NO: 4, and a sequencing primer including the sequence of SEQ ID NO: 7.

The CpG islands located at positions -242 and -236 from the transcription initiation site (+1) in the sequence of the *ABCC9* gene SEQ ID NO: 2 may be amplified.

Particularly, PCR primers for measuring methylation of the *ABCC9* gene may include primers including the sequence of SEQ ID NO: 5 and the sequence of SEQ ID NO: 6, and a sequencing primer including the sequence of SEQ ID NO: 8.

| Gene | Primer | No . | Sequence (5'→3') | CpG position * | Amplicon size (bp) |
|---|---|---|---|---|---|
| *PRRX* 1 | Forward | 3 | **TGGGGAGATTGATTAGTTTTAAGG** | -1, 361, -1,355, -1,351, -1,348 | 175 |
| | Reverse | 4 | **Biotin-AAATTTCCAACTCAACTACCTCC** | | |
| | Sequencing | 7 | **TTTAGTATAGTAGATGGTA** | | |
| *ABCC 9* | Forward | 5 | **GTGGGTGTTTTTATTTAGAATGAGTTT** | -242, - 236 | 113 |
| | Reverse | 6 | **Biotin-ACTCTATAAACACCCCTTTATTAAC** | | |
| | Sequencing | 8 | **AGAATGAGTTTTTTATTTT** | | |

| | | | | | |
|---|---|---|---|---|---|
| *Distance (nucleotide) from transcription initiation point (+1): Position of CpG site on genomic DNA used for methylation measurement | | | | | |

Among the primers, the reverse primer may additionally include a marker.

The marker included in the reverse primer may be, but not limited to, biotin, FAM, Cy5, Cy3, FITC, EDANS (5-(2'-aminoethyl)amino-1-naphthalene sulfate), tetramethylrhodamine (TMR), tetramethylrhodamine isocyanate (TMRITC), x-rhodamine, DIG, or antibodies or nanoparticles conjugated thereto.

Here, by reacting with a binder that induces color development of the marker, it is possible to confirm whether color development or fluorescence is expressed and to detect amplification of the target nucleic acid. As such, the binder may be, but not limited to, streptavidin.

In one embodiment, CpG island methylation may be detected by quantitative methylation-specific real-time PCR (qMSP) . Primers and probes may be used for methylation analysis through qMSP.

The primers may be used simultaneously, for example, by pairing forward and reverse primers. A forward primer that specifically amplifies the methylated *PRRX1* gene may include, for example, the sequence of SEQ ID NO: 9. A reverse primer may include, for example, the sequence of SEQ ID NO: 10. The primers specifically amplifying the methylated *PRRX1* gene may include a primer pair of SEQ ID NOs: 9 and 10.

A forward primer that specifically amplifies the methylated *ABCC9* gene may include, for example, the sequence of SEQ ID NO: 11. A reverse primer may include, for example, the sequence of SEQ ID NO: 12. The primers specifically amplifying the methylated *ABCC9* gene may include a primer pair of SEQ ID NOs: 11 and 12.

Among the primers, the reverse primer may additionally include a marker.

The marker included in the reverse primer may be, but not limited to, biotin, FAM, Cy5, Cy3, FITC, EDANS (5-(2'-aminoethyl)amino-1-naphthalene sulfate), tetramethylrhodamine (TMR), tetramethylrhodamine isocyanate (TMRITC), x-rhodamine, DIG, or antibodies or nanoparticles conjugated thereto.

Here, by reacting with a binder that induces color development of the marker, it is possible to confirm whether color development or fluorescence is expressed and to detect amplification of the target nucleic acid. As such, the binder may be, but not limited to, streptavidin.

A step of performing treatment with a probe capable of complementary hybridization to the methylated *PRRX1* or *ABCC9* gene specifically amplified by the primers may be further included.

In hybridization reaction, the conditions used to achieve a certain level of stringency vary depending on the nature of the nucleic acid to be hybridized. For example, the length of the nucleic acid site to be hybridized, the degree of homology, the nucleotide sequence composition (e.g., GC/AT composition ratio), and the type of nucleic acid (e.g., RNA, DNA) are considered in selecting the hybridization conditions. A further consideration is whether the nucleic acid is immobilized, for example on a filter or the like.

Examples of very stringent conditions are as follows: 2X SSC/0.1% SDS at room temperature (hybridization conditions); 0.2X SSC/0.1% SDS at room temperature (low stringency conditions); 0.2X SSC/0.1% SDS at 42°C (moderate stringency conditions); and 0.1X SSC at 68°C (high stringency conditions). The washing process may be performed using any one of these conditions, and for example, high stringency conditions or each of the above conditions may be used, the conditions may be applied for 10 to 15 minutes each in the order described above, or all or some of the conditions described above may be repeatedly applied. As described above, however, the optimal conditions vary depending on the special hybridization reaction involved, and may be determined through experimentation. Generally, high stringency conditions are used for hybridization of the probe of interest.

A probe capable of complementary hybridization to the amplified methylated *PRRX1* or *ABCC9* gene may include, for example, the sequence of SEQ ID NO: 13 or 14. A probe capable of complementary hybridization to the amplified methylated *PRRX1* gene may include the sequence of SEQ ID NO: 13. A probe capable of complementary hybridization to the amplified methylated *ABCC9* gene may include the sequence of SEQ ID NO: 14.

Particularly, the primers and probes for detecting methylation of the *PRRX1* gene and/or *ABCC9* gene according to the present invention are described below.

| Gene | Primer | No . | Primer sequence (5'→3') | Amplico n size (bp) |
|---|---|---|---|---|
| *PRRX 1* | Forward | 9 | **CGGTTTATATCGAATTGGATGTC** | 124 |
| | Reverse | 10 | AAAGATTCGGCGACCACCGACGAAAACCTAAACTAAACGCCGCA CG | |
| | Probe | 13 | **/56-FAM/CGTTATTAGATTGCGTTTAGTATAGTAGATG/3SFCQ1/** | |
| *ABCC 9* | Forward | 11 | **GACGGTGGGTAAGGGTCG** | 113 |
| | Reverse | 12 | AAAGATTCGGCGACCACCGACTAATTTTCCGCTTTTCCCCGACG ACCTA | |
| | Probe | 14 | **/5HEX/CGATAGCGCGGCGGTTGGT/3SFCQ1/** | |

In some cases, at least one reagent that differently modifies the methylated *PRRX1* gene and/or *ABCC9* gene and the unmethylated *PRRX1* gene and/or *ABCC9* gene may be additionally included.

At least one reagent that modifies the methylated DNA and the unmethylated DNA differently from each other may be used without limitation, so long as it is able to distinguish between unmethylated cytosine and methylated cytosine, and examples thereof may include bisulfite, hydrogen sulfite, disulfite, and combinations thereof, but are not limited thereto. Particularly, when using the reagent described above, methylated cytosine is not converted, and unmethylated cytosine may be converted into uracil or a base other than cytosine.

When genomic DNA is treated with bisulfite, cytosine in the 5'-CpG-3' region remains as cytosine in case of methylation, and is converted into uracil or a base other than cytosine in case of non-methylation. Therefore, primers corresponding to a region where the 5'-CpG-3' sequence exists may be constructed for the sequence converted after treatment with bisulfite.

A nucleic acid isolated from a specimen is obtained from a biological sample of the specimen. In order to diagnose lung cancer or the stage of progression of lung cancer, the nucleic acid has to be isolated from lung tissue by scraping or biopsy. Such a sample may be obtained by various medical procedures known in the art.

The extent of methylation of the nucleic acid in the sample obtained from the specimen is measured through comparison with the same portion of the nucleic acid from a specimen without cell growth abnormality in lung tissue. Hypermethylation indicates the presence of a methylated allele in at least one nucleic acid. When the same nucleic acid is tested in a specimen without cell growth abnormality in lung tissue, the methylated allele does not appear.

The present invention is based on the discovery of the relationship between lung cancer and hypermethylation of *PRRX1* (paired related homeobox 1) and/or *ABCC9* (ATP binding cassette subfamily C member 9) genes.

"Normal" cells are cells that do not show abnormal cell morphology or a change in cytological properties. "Tumor" cells are cancer cells, and "non-tumor" cells are cells that are part of the diseased tissue but are not considered to be the site of tumor.

According to the present invention, early diagnosis of cell growth abnormality in lung tissue of a specimen is possible by determining the methylation stage of at least one nucleic acid isolated from the specimen. The methylation stage of at least one nucleic acid may be compared with the methylation status of at least one nucleic acid isolated from a specimen without cell growth abnormality in lung tissue. It is preferred that the nucleic acid be a nucleic acid containing CpG, such as a CpG island.

According to the present invention, it is possible to diagnose a predisposition to cell growth abnormality in lung tissue of a specimen, including determining methylation of at least one nucleic acid isolated from the specimen. The methylation stage of at least one nucleic acid may be compared with the methylation status of at least one nucleic acid isolated from a specimen having no predisposition to cell growth abnormality in lung tissue.

Herein, the term "predisposition" refers to a property that is susceptible to the cell growth abnormality. A specimen having a predisposition is one which does not yet exhibit cell growth abnormality but in which cell growth abnormality is present or the likelihood of developing cell growth abnormality is increased.

The presence of CpG methylation in the target DNA may be an indicator of lung cancer, and for example, CpG methylation in the promoter region of the target DNA may be measured.

The CpG-containing gene is typically DNA. However, the method of the present invention may be performed using a sample containing, for example, DNA, or DNA and RNA including mRNA, in which the DNA or RNA may be single-stranded or double-stranded, or a sample containing a DNA-RNA hybrid may be used.

A mixture of nucleic acids may also be used. As used herein, the term "multiple" includes both the case in which there is a plurality of specific nucleic acid sequence sites to be detected in a kind of gene and the case in which a plurality of target DNA sequences is included in a single tube (single reactor). The specific nucleic acid sequence to be detected may be a fraction of a large molecule, or may be present initially in the form of a discrete molecule in which the specific sequence constitutes the entire nucleic acid sequence. The nucleic acid sequence need not be a nucleic acid present in a pure form, and the nucleic acid may be a minor fraction of a complex mixture, such as one contained in whole human DNA.

Particularly, the present invention is directed to detecting methylation of a plurality of target DNA sequences in a sample in a single reactor, in which the sample may include multiple target DNA sequences, and any target DNA may be used without limitation so long as it is a gene that affects the development or progression of lung cancer when the expression thereof is suppressed due to abnormal methylation, as well as a control gene.

As used herein, the terms "sample," "clinical sample," and "specimen" refer to a broad range of bodily fluids, including any biological fluid obtained from an individual, bodily fluid, cell line, tissue culture, etc., depending on the type of analysis being performed. Methods of obtaining bodily fluid and tissue biopsies from mammals are typically well known.

In the present invention, the sample may be derived from a human body, and for example, the sample may include lung cancer tissue, cells, stool, urine, blood, serum, respiratory tract lavage fluid, such as bronchial lavage fluid, or plasma.

With regard to the primers used in the present invention, when treated with the reagent, for example, bisulfite in step (a), cytosine in the 5'-CpG-3' region remains as cytosine in case of methylation, and is converted into uracil in case of non-methylation. Therefore, for example, primers corresponding to a region where the 5'-CpG-3' sequence exists may be constructed for the sequence converted after treatment with the reagent, for example bisulfite.

The primer may be constructed to have "substantial" complementarity with each strand of the gene locus to be amplified. This means that the primer has sufficient complementarity to hybridize with the corresponding nucleic acid strand under conditions for performing polymerization reaction.

Still another aspect of the present invention relates to a kit for detecting methylation of target DNA including the composition described above.

In one embodiment, the kit may include compartmentalized carrier means for holding a sample, a container including a reagent, and a container including a substance capable of detecting CpG island methylation of a *PRRX1* gene and/or a substance capable of detecting CpG island methylation of an *ABCC9* gene. In some cases, a container including a probe for detecting each of the methylated *PRRX1* gene and/or methylated *ABCC9* gene amplicons may be further included.

The carrier means is suitable for accommodating one or more containers, such as bottles or tubes, each container containing independent components for use in the method of the present invention. In the context of the present invention, a person of ordinary skill in the art may readily determine the apportionment of the necessary agents in the containers.

According to the present invention, lung cancer may be diagnosed by examining methylation of the marker gene *PRRX1* or *ABCC9.*

The possibility of progression to lung cancer may be diagnosed by examining methylation of the marker gene *PRRX1* or *ABCC9* using a sample that exhibits a normal phenotype.

"Cell transformation" refers to a change in the characteristics of a cell from one type to another, such as from normal to abnormal, from non-cancerous to cancerous, from undifferentiated to differentiated, or from a stem cell to a non-stem cell. Furthermore, the transformation may be recognized by the morphology, phenotype, biochemical properties, etc. of the cells.

Herein, "early detection" of cancer means discovering the likelihood of cancer before metastasis, preferably before observation of a morphological change in the tissue or cells of a specimen. Furthermore, "early detection" of cell transformation means high probability of a cell to undergo transformation at an early stage before the cell is morphologically designated as being transformed.

In the present invention, the *PRRX1* or *ABCC9* gene may be used alone or in combination as a diagnostic or predictive marker for lung cancer. When the *PRRX1* and *ABCC9* genes are used in combination, they may be used in a panel display form. As such, depending on the number of genes methylated together and importance thereof, genes may be ranked and weighted, and the level of likelihood of developing into cancer may be assigned.

A better understanding of the present invention may be obtained through the following examples. These examples are merely set forth to illustrate the present invention and are not to be construed as limiting the scope of the present invention, as will be apparent to those skilled in the art.

### Example 1: Discovery of lung cancer-specific methylation genes

To select biomarkers specifically methylated in lung cancer, 500 ng of genomic DNA from cancer tissues obtained from surgical tissues of 13 lung cancer patients (stage I-III, provided by Chungnam National University Hospital) and normally appearing tissues adjacent thereto was sonicated (Vibra Cell, SONICS), producing genomic DNA fragments of about 200-300 bp.

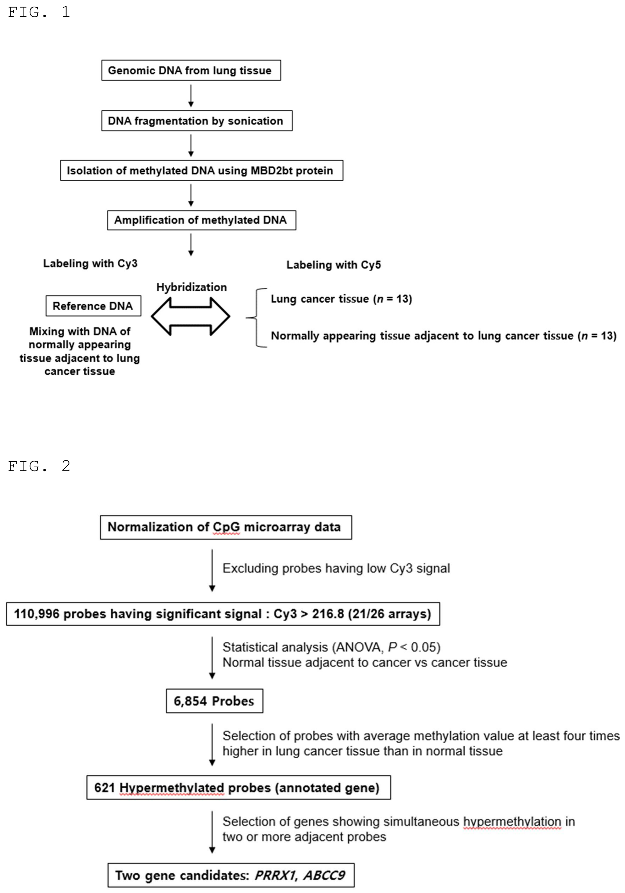

Only methylated DNA was obtained from genomic DNA using methyl binding domain (MBD), known to bind to methylated DNA, according to the method described in Oh et al. (J. Mol. Diag. 2013;15(4):498-507). The methylated DNA thus obtained, namely methylated DNA bound to MBD2bt obtained according to the method described in Oh et al (J. Mol. Diag. 2013;15(4):498-507) was amplified and then hybridized to a human 244K human CpG microarray (Agilent, USA) (FIG. 1). After hybridization, a series of washing processes was performed followed by scanning using a laser scanner (Agilent, USA). The signal values from the microarray images were determined using the Feature Extraction program v.9.5.3.1 (Agilent, USA), calculating the relative intensity difference of signals between lung cancer tissues of lung cancer patients and adjacent normally appearing tissue samples.

To select probes with reliable hybridization signals, the GeneSpring 7.3.1 program (Agilent, USA) was used. To select probes specifically hypermethylated in lung cancer, an ANOVA test was performed by comparing lung cancer tissues and normally appearing tissues adjacent to lung cancer, and 6,854 probes with a P value of 0.05 or less were selected. Furthermore, 621 probes that were hypermethylated by at least four times on average in lung cancer tissues compared to normally appearing tissues were selected again, and among the genes that simultaneously showed hypermethylation in two or more adjacent probes in the promoter region, two novel biomarker candidate genes (*PRRX1* and *ABCC9*) in which methylation had not been reported in lung cancer were selected (FIG. 2).

The two biomarker candidate genes analyzed using the method described above are shown in Table 1 below. In addition, the sequence of the region corresponding to each probe of these two genes showing hypermethylation in the CpG microarray analysis was analyzed using MethPrimer (http://itsa.ucsf.edu/~urolab/methprimer/index1.html), confirming the presence of CpG islands.

**[Table 1] List of methylation biomarker candidate genes for lung cancer diagnosis**

| Candidate gene | Probe position* | GenBank No. | Description |
|---|---|---|---|
| *PRRX1* | -1,505, -1,411, -1,343 | NG_031856 | Paired related homeobox 1 |
| *ABCC9* | -373, -264 | NG_012819 | ATP binding cassette subfamily C member 9 |

| | | | |
|---|---|---|---|
| *Sequence distance (bp) from transcription initiation site (+1) | | | |

### Example 2: Confirmation of methylation of two biomarker candidate genes in lung cancer cell line

To further confirm the methylation status of the biomarker candidate genes selected in Example 1, pyrosequencing was performed on each promoter region.

To convert unmethylated cytosine into uracil using bisulfite, total genomic DNA was isolated from the lung cancer cell line H358 (Korea Cell Line Bank, KCLB No. 25807), and 200 ng of the genomic DNA was treated with bisulfite using the EZ DNA methylation-Gold kit (Zymo Research, USA). When DNA is treated with bisulfite, unmethylated cytosine is converted into uracil, and methylated cytosine remains unchanged. The bisulfite-treated DNA was eluted with 20 µl of sterile distilled water and pyrosequencing was performed.

PCR and sequencing primers for pyrosequencing of the two genes were designed using PyroMark Assay Design 2.0 (QIAGEN, Germany). PCR and sequencing primers for measuring methylation of each gene are listed in Table 2 below.

**[Table 2] Bisulfite PCR and pyrosequencing primers**

| Gene | Primer | Sequence (5'→3') | CpG position * | Amplico n size (bp) |
|---|---|---|---|---|
| *PRRX 1* | Forward | **TGGGGAGATTGATTAGTTTTAAGG** | -1, 361, -1, 355, -1, 351, -1, 348 | 175 |
| | Reverse | **Biotin-AAATTTCCAACTCAACTACCTCC** | | |
| | Sequencing | **TTTAGTATAGTAGATGGTA** | | |
| *ABCC 9* | Forward | **GTGGGTGTTTTTATTTAGAATGAGTTT** | -242, - 236 | 113 |
| | Reverse | Biotin- ACTCTATAAACACCCCTTTATTAAC | | |
| | Sequencing | **AGAATGAGTTTTTTATTTT** | | |

| | | | | |
|---|---|---|---|---|
| *Distance (nucleotide) from transcription initiation point (+1): Position of CpG site on genomic DNA used for methylation measurement | | | | |

20 ng of the bisulfite-converted genomic DNA was amplified by PCR. A PCR solution (20 ng of bisulfite-converted genomic DNA, TOPsimple^{™} DryMIX-HOT (Enzynomics, P581H, Korea), 2 µl (10 pmol) of PCR primers) was treated at 95°C for 5 minutes, followed by a total of 45 cycles of 95°C for 30 seconds, 60°C for 30 seconds, and 72°C for 30 seconds, and then reacted at 72°C for 5 minutes. Amplification of the PCR product was confirmed by electrophoresis using 2.0% agarose gel.

After treating the amplified PCR product with PyroMark Q48 Advanced CpG Reagents (QIAGEN, Germany), pyrosequencing was performed using the PyroMark Q48 Autoprep system (QIAGEN, Germany). After pyrosequencing, the extent of methylation was measured by calculating the methylation index. The methylation index was calculated by determining the average rate of cytosine binding to each CpG site.

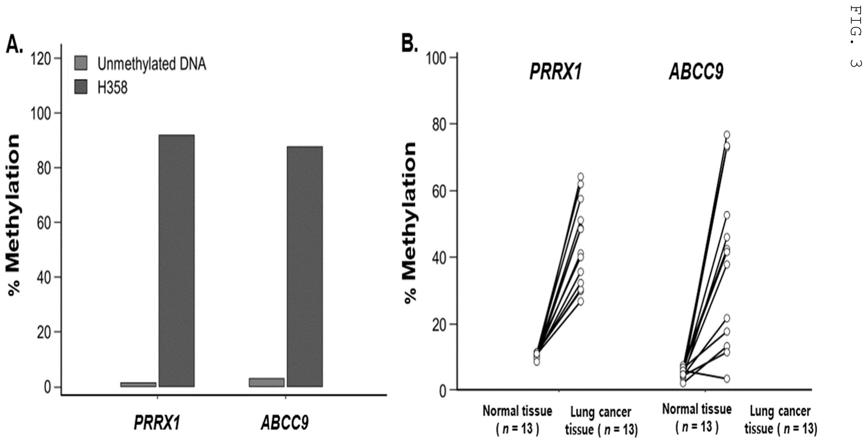

Based on results of quantitative measurement of the extent of methylation of the biomarker candidate genes in the lung cancer cell line using the pyrosequencing method, as shown in FIG. 3A, methylation of both biomarker genes at a very high level of 80% or more in the H358 cell line was confirmed. The two genes showed high methylation levels in the lung cancer cell line, indicating potential utility thereof as biomarkers for lung cancer diagnosis. Accordingly, an additional methylation verification experiment using tissue samples was conducted as described below.

### Example 3: Measurement of methylation of biomarker genes in lung cancer patient cancer tissue and adjacent normally appearing tissue

For the two novel biomarker candidate genes of Example 1 to be useful as diagnostic markers for lung cancer, they should exhibit low methylation levels in normal lung tissue but high methylation levels in lung cancer tissue.

Therefore, usefulness of the two genes as biomarkers was verified using lung cancer patient tissues. To this end, DNA from the surgical tissues (lung cancer tissue and adjacent normally appearing tissue) of 13 lung cancer patients used in the microarray experiment in Example 1 was treated with bisulfite, eluted with 20 µl of sterile distilled water, and used for pyrosequencing.

20 ng of the bisulfite-converted genomic DNA was amplified by PCR. A PCR solution (20 ng of bisulfite-converted genomic DNA, TOPsimple^{™} DryMIX-HOT (Enzynomics, P581H, Korea), 2 µl (10 pmol) of PCR primers) was treated at 95°C for 5 minutes, followed by a total of 45 cycles of 95°C for 30 seconds, 60°C for 30 seconds, and 72°C for 30 seconds, and then reacted at 72°C for 5 minutes. Amplification of the PCR product was confirmed by electrophoresis using 2.0% agarose gel.

Pyrosequencing was performed in the same manner as in Example 2.

Thereby, as shown in FIG. 3B, both genes were confirmed to show a higher level of methylation in lung cancer tissues than in normally appearing tissues. *PRRX1* showed a higher methylation level in lung cancer tissues of 13 out of 13 patients (100%) and *ABCC9* showed a higher methylation level in lung cancer tissues of 12 out of 13 patients (92.3%) than in adjacent normally appearing tissues.

Table 3 below shows the average methylation values of the *PRRX1* and *ABCC9* genes in lung cancer tissues and adjacent normally appearing tissues. To determine whether there was a statistically significant difference in the methylation levels between lung cancer tissues and normally appearing tissues, a chi-squared test was performed, and the results showed that the P value was 0.0001 or less, indicating a high level of significance (Table 3).

**[Table 3] Results of quantitative methylation analysis of novel biomarkers PRRX1 and ABCC9**

| Gene | Average methylation level (%, mean±standard deviation) | | |
|---|---|---|---|
| | Normally appearing tissue | Lung cancer tissue | *P* value* |
| *PRRX1* | **10.6 ± 08** | **437 ± 12.6** | **< 00001** |
| *ABCC9* | **5.2 ± 1.5** | **39.3 ± 24.9** | **= 0.0001** |

| | | | |
|---|---|---|---|
| *P value obtained from chi-squared test results | | | |

### Example 4: Evaluation of ability of novel biomarker genes PRRX1 and ABCC9 to diagnose lung cancer in bronchial lavage fluid

To further evaluate the ability of the two candidate genes to diagnose lung cancer, methylation was analyzed in bronchial lavage fluids from lung cancer patients and non-lung-cancer benign lung disease patients.

For methylation analysis, a quantitative methylation-specific real-time PCR (qMSP) technique was established, and primer and probe sequences used for methylation analysis are shown in Table 4 below.

**[Table 4] Primer and probe sequences for qMSP of PRRX1 and ABCC9 genes**

| Gene | Primer | Primer sequence (5'→3') | Amplicon size (bp) |
|---|---|---|---|
| *PRRX1* | Forward | **CGGTTTATATCGAATTGGATGTC** | 124 |
| | Reverse | AAAGATTCGGCGACCACCGACGAAAACCTAAACTAAACGCC GCACG. | |
| | Probe | **/56-FAM/CGTTATTAGATTGCGTTTAGTATAGTAGATG/3SFCQ1/** | |
| *ABCC9* | Forward | **GACGGTGGGTAAGGGTCG** | 113 |
| | Reverse | AAAGATTCGGCGACCACCGACTAATTTTCCGCTTTTCCCCGA CGACCTA. | |
| | Probe | **/5HEX/CGATAGCGCGGCGGTTGGT/3SFCQ1/** | |

After confirming methylation in the lung cancer cell line, methylation was measured in DNA from bronchial lavage fluid using the relevant technique. Genomic DNA was isolated from bronchial lavage fluids of 20 non-lung-cancer patients and 20 lung cancer patients (provided by Konyang University Hospital), after which 40 ng of the isolated genomic DNA was treated with bisulfite using the EZ DNA methylation-Gold kit (Zymo Research, USA) and eluted with 10 µl of sterile distilled water, and qMSP was performed. The PCR conditions and reaction solution composition are shown in Table 5 for the bronchial lavage fluid, and the results of PCR were analyzed after treatment with 35-ΔC_{T} (biomarker gene C_{T} - control gene C_{T}) values for normalization.

**[Table 5] qMSP conditions and reaction solution composition**

| PCR conditions | Temperature | Time | Cycle |
|---|---|---|---|
| | 95°C | 5 min | 1 cycle |
| | 95°C | 15 sec | 5 cycles |
| | 70°C | 45 sec | |
| | 95°C | 15 sec | 35 cycles |
| | 60°C | 45 sec | |

| | Items | | Concentration |
|---|---|---|---|
| | P5 (Universal primer) | | 5 pmol |
| | Biomarker gene | Forward | 5 pmol |
| | | P5-Reverse | 1 pmol |
| | | Probe | 5 pmol |
| PCR solution composition | COL2A1 (Control gene) | Forward | 2.5 pmol |
| | | Reverse | 2.5 pmol |
| | | Probe | 2.5 pmol |
| | TOPreal^{™} Fast qPCR 5X PreMIX TaqManProbe* | | 5 µl |
| | Template** | | 5 µl |
| | Up to DW | | 25 µl |
| | Total | | 25 µl |

| | | | |
|---|---|---|---|
| *Enzynomics, Korea, Cat No. GT-RT650 (Customized) **20 ng of bisulfite-treated genomic DNA | | | |

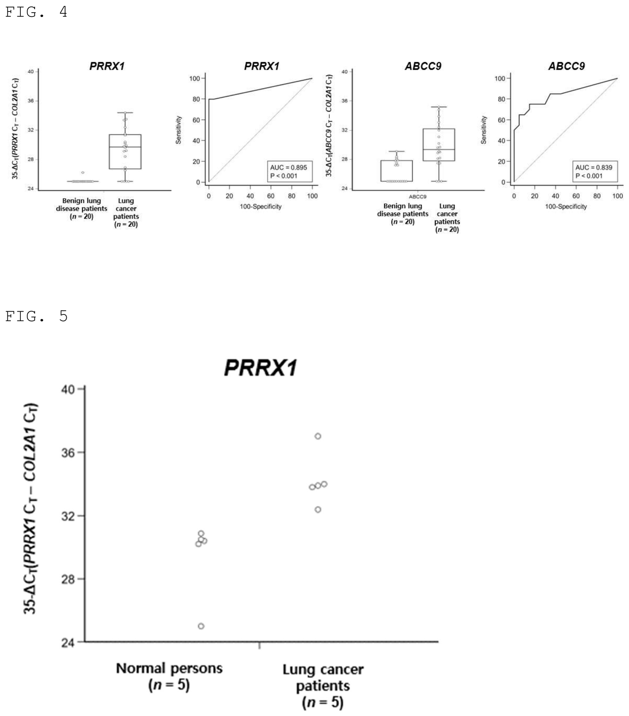

Based on results of measurement of methylation of the two biomarker candidates in bronchial lavage fluid, methylation was higher in lung cancer patients than in non-lung-cancer patients (FIG. 4). To evaluate the ability of these two genes to diagnose lung cancer, ROC (receiver operating characteristics) curve analysis was performed, the optimal cut-off for lung cancer diagnosis was set, and sensitivity and specificity were evaluated (FIG. 4). Thereby, the *PRRX1* gene had a sensitivity of 80% (16/20) and a specificity of 100% (20/20) for lung cancer diagnosis (Table 6). The *ABCC9* gene had a sensitivity of 75% (15/20) and a specificity of 80% (16/20) for lung cancer diagnosis (Table 6).

**[Table 6] Analysis of qMSP results of bronchial lavage fluid**

| Gene | *PRRX1* | *ABCC9* |
|---|---|---|
| Cut-off | >26.5 | >28.0 |
| Sensitivity (n=20) | 80% (16/20) | 75% (15/20) |
| Specificity (n=20) | 100% (20/20) | 80% (17/20) |

In conclusion, among these two genes, *PRRX1* was confirmed to be useful for lung cancer diagnosis due to excellent sensitivity and specificity thereof, and was determined as the final biomarker.

### Example 5: Evaluation of ability of novel biomarker gene PRRX1 to diagnose lung cancer in blood

The ability of the novel biomarker *PRRX1* to diagnose lung cancer in plasma was evaluated using the same technique as Example 4. To this end, DNA was isolated from 1.0 ml of plasma from 5 normal persons (provided by Eulji University Hospital) and 5 lung cancer patients (provided by Chungnam National University Hospital), treated with bisulfite using the EZ DNA methylation-Gold kit (Zymo Research, USA), and then eluted with 5 µl of sterile distilled water, and qMSP was performed in the same manner as in Example 4 and methylation was measured.

Thereby, methylation was confirmed to be higher in lung cancer patients than in normal persons (FIG. 5). These results showed that the novel biomarker gene *PRRX1* was also useful for lung cancer diagnosis using the blood.

### [Industrial Applicability]

According to the present invention, the *PRRX1* or *ABCC9* gene is provided as a lung cancer-specific methylation marker, and methylation is detected using the *PRRX1* or *ABCC9* gene as a biomarker, thereby making it possible to detect methylation with high detection sensitivity, ultimately enabling useful diagnosis of lung cancer through accurate and rapid lung cancer detection.

Having described certain parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### [sequence List Free Text]

An electronic file is attached.

## Claims

1. A composition for diagnosing lung cancer containing a substance capable of detecting CpG island methylation of a *PRRX1* (paired related homeobox 1) gene and/or an *ABCC9* (ATP binding cassette subfamily C member 9) gene.

2. The composition according to claim 1, wherein the CpG island is located in a promoter of the *PRRX1* or *ABCC9* gene.

3. The composition according to claim 1, wherein the CpG island of the *PRRX1* gene is present at a position - 1,505 to -1,299 from a transcription initiation site in a sequence of SEQ ID NO: 1.

4. The composition according to claim 1, wherein the CpG island of the *ABCC9* gene is present at a position -373 to -220 from a transcription initiation site in a sequence of SEQ ID NO: 2.

5. The composition according to claim 1, wherein the substance capable of detecting CpG island methylation is a primer or primer pair capable of amplifying a fragment comprising a methylated CpG island, a probe capable of hybridizing with the methylated CpG island, a sequencing primer, a sequencing-by-synthesis primer, or a sequencing-by-ligation primer.

6. The composition according to claim 1, wherein the substance capable of detecting CpG island methylation is at least one primer selected from the group consisting of sequences of SEQ ID NOs: 3 to 12 or a probe comprising a sequence of SEQ ID NO: 13 or 14.

7. The composition according to claim 1, further comprising at least one reagent that differently modifies methylated *PRRX1* and/or *ABCC9* and unmethylated *PRRX1* and/or *ABCC9.*

8. The composition according to claim 7, wherein the reagent is bisulfite, hydrogen sulfite, disulfite, or a combination thereof.

9. A kit for diagnosing lung cancer comprising the composition according to any one of claims 1 to 8.

10. A method of providing information for lung cancer diagnosis, comprising treating a sample with a substance capable of detecting CpG island methylation of a *PRRX1* (paired related homeobox 1) gene and/or an *ABCC9* (ATP binding cassette subfamily C member 9) gene.

11. The method according to claim 10, wherein the CpG island is located in a promoter of the *PRRX1* or *ABCC9* gene.

12. The method according to claim 10, wherein the CpG island of the *PRRX1* gene is present at a position - 1,505 to -1,229 from a transcription initiation site in a sequence of SEQ ID NO: 1.

13. The method according to claim 10, wherein the CpG island of the *ABCC9* gene is present at a position -373 to -220 from a transcription initiation site in a sequence of SEQ ID NO: 2.

14. The method according to claim 10, wherein the substance capable of detecting CpG island methylation is a primer or primer pair capable of amplifying a fragment comprising a methylated CpG island, a probe capable of hybridizing with the methylated CpG island, a sequencing primer, a sequencing-by-synthesis primer, or a sequencing-by-ligation primer.

15. The method according to claim 10, wherein the substance capable of detecting CpG island methylation is at least one primer selected from the group consisting of sequences of SEQ ID NOs: 3 to 12 or a probe comprising a sequence of SEQ ID NO: 13 or 14.

16. The method according to claim 10, further comprising performing treatment with at least one reagent that differently modifies methylated *PRRX1* and/or *ABCC9* and unmethylated *PRRX1* and/or *ABCC9.*

17. The method according to claim 16, wherein the reagent is bisulfite, hydrogen sulfite, disulfite, or a combination thereof.
